# EUROPÄISCHE PATENTSCHRIFT

(11) **EP 3 291 746 B1**
(45) Veröffentlichungstag und Bekanntmachung des Hinweises auf die Patenterteilung: **05.02.2020**
(21) Anmeldenummer: 16731313.9
(22) Anmeldetag: 09.05.2016
(51) Int. Cl.: A61B 17/12

(54) **NASENHÖHLEN- ODER NASENNEBENHÖHLENTAMPONADE**
TAMPONADE FOR NASAL CAVITIES OR SINUS CAVITIES
TAMPONNEMENT DES CAVITÉS NASALES OU DES SINUS

(30) Priorität: 07.05.2015 DE 102015005725; 02.11.2015 DE 102015014153
(43) Veröffentlichungstag der Anmeldung: 14.03.2018
(73) Patentinhaber: Creative Balloons GmbH, 68753 Waghäusel (DE)
(72) Erfinder: GÖBEL, Fred, 69259 Wilhelmsfeld (DE)
(74) Vertreter: Küchler, Stefan
(86) Internationale Anmeldenummer: PCT/IB2016/000610
(87) Internationale Veröffentlichungsnummer: WO 2016/178080

(56) Entgegenhaltungen:
- DE-A1- 3 712 502
- DE-A1- 4 010 975
- DE-U1- 20 320 631
- GB-A- 2 258 811
- US-B1- 6 607 546

## Beschreibung

Die vorliegende Erfindung richtet sich auf eine Nasenhöhlen- oder Nasennebenhöhlentamponade zur krafthomogen wirkenden Tamponade einer Kavität des Nasentraktes im Bereich eines zu der Kavität führenden Ostiums.

Bei der operativen Versorgung bzw. operativen Eröffnung partiell oder vollständig verschlossener Zugangsöffnungen (Ostien) zu den sogenannten Nasennebenhöhlen (para-nasale Sinus) kommt es in vielen Fällen im Rahmen der anschließenden Wundheilung zu Wiederverschlüssen des erweiterten Ostiums. Die sich postoperativ durch Bildung von Granulations- und Narbengewebe entwickelnde Engstellung des Ostiums kann so ausgeprägt sein, dass der Erfolg der operativen Weitung bereits nach wenigen Wochen weitgehend oder auch vollständig verloren geht.

Zur Vermeidung derartiger, mit der Wundheilung einhergehender Re-Stenosierung werden von vielen Operateuren tamponierende Körper in den operativ erweiterten Übergang der Nasenhaupthöhle in die sich anschließende Nasennebenhöhle eingelegt. Die tamponierenden Elemente bestehen heute in der Mehrzahl aus fingerlingartigen Hüllen, in die ein sich elastisch verformbares, schwämmchenartiges Element eingesteckt ist, das die umschließende glattwandige Hülle des Fingerlings unter moderatem Druck an die Wundflächen schmiegt.

Ebenfalls noch in der klinischen Praxis etabliert, ist die Einlage eines gazeartigen Streifens, mit dem das Ostium selbst sowie der Bereich vor und hinter dem Ostium ausgestopft wird.

Problematisch sind bei diesen Techniken vor allem die Schmerzen bei der Entnahme des tamponierenden Materials sowie die in der Regel mit der Entnahme einhergehende Blutung. Bei Liegezeiten von bis zu einer Woche und darüber hinaus kann es zu erheblichen Verkrustungen bzw. Anheftungen des Tamponadematerials mit den Wundflächen kommen, die bei der Mobilisation der Tamponade die Wundflächen aufreißen und so wiederum zu überschießender Neubildung bzw. Granulation von Gewebe führen kann.

Seit einigen Jahren werden für die post-operative Tamponade der Nasenhöhle und der Nasennebenhöhlen Tamponadesysteme auf Basis aufblasbarer Ballonkörper angeboten. Diese bestehen in der Regel aus vorgeformten Ballonkomponenten, die einem schlauch- oder rohrartigen Schaftkörper aufsitzen. Die Ballons sind in der Regel aus Silikon oder PVC gefertigt, entsprechend dickwandig in ihrer Ausführung und im Falle von Silikon in der Regel mit der Option einer gewissen elastischen Aufdehnung des Hüllenkörpers unter Beaufschlagung von Fülldruck ausgestattet. Der tragende Schaft besteht in der Regel aus relativ steifem Material um erlaubt die gerichtete Führung der Tamponade durch den Operateur. Bei entsprechenden Ballontamponaden zur Versorgung der Nasennebenhöhlen sind die tamponierenden Ballonkomponenten in einigen Fällen im Bereich des innerhalb des Ostiums zu platzierenden Segmentes tallienartig verengt, um dort eine ausreichende Fixierung des die Nebenhöhle ausfüllenden Ballonkörpers zu erreichen. Es sind zudem Ballontamponaden bekannt, die mit besonderen, netzartigen Überzügen aus zelluloseartigen Materialien ausgestattet sind, die eine lokal blutungsstillende Wirkung haben.

Der DE 203 20 631 U1 ist eine Vorrichtung zur Verwendung bei Heilprozessen zu entnehmen, die aus einem flexiblen, doppelwandigen, inflatierbaren Schlauchsegment besteht. Eine Einrichtung zur Fixierung der Tamponade innerhalb einer Kavität ist dort jedoch nicht offenbart.

Die US 6,607,546 B1 offenbart einen Nasenkatheter, der nicht nur einen Ballon aufweist, sondern mehrere Ballone, und ist demzufolge für die Anwendung sehr komplex.

Bei der DE 37 12 502 A1 wird im Rahmen einer Nasentamponade die Kavität nicht durch einen Ballon tamponiert; vielmehr dient ein dort vorhandender Ballon ausschließlich als Verschlusselement.

Die DE 40 10 975 A1 schlägt zur Fixierung eines Andruckballons eine Klammer vor, die an der Nasenscheidewand festlegbar ist.

Die GB 2 258 811 A sieht einen intranasalen Latexballon mit einem Rückhaltekragen auf, der nicht Bestandteil des Ballons ist, sondern im Bereich einer Zuleitung desselben angeordnet ist.

Problematisch bei den beschriebenen Vorrichtungen ist vor allem die unzureichende Fähigkeit des Ballons zur flächigen Anschmiegung an die individuelle Ausformung der Nebenhöhle bzw. deren jeweilige Oberfläche. Insbesondere kann durch die bekannten Tamponadetechniken keine ausreichend durchgängig flächige Schmiegung des Ballonhüllenmaterials an die für den Tamponadeerfolg kritischen Wundflächen im Bereich des Ostiums selbst erreicht werden.

Die Art der Schmiegung der Ballonhülle an die jeweilige Wundfläche ist vor allem dann von Relevanz, wenn in den zu tamponierenden Raum hineinreichende prominente Strukturen zur Ausbildung von Druckspitzen führen, die die lokale Durchblutung zum Erliegen bringen und so den Prozess der Wundheilung stören und verzögern können. Wegen der beschriebenen inhomogenen Kraftentwicklung auf die anliegenden Gewebe ermöglichen herkömmliche Ballontamponaden keine ausreichend Kontrolle bzw. Steuerung der jeweils wirksamen Tamponadedrucke durch den Operateur.

Ein weiteres wesentliches Problem bei der konventionellen Tamponade von Nasennebenhöhlenostien besteht in der in der Regel vollständigen Einschränkung der freien Nasenatmung des Patienten.

Ferner kann bei der Mehrzahl der Tamponaden keine ausreichende Belüftung der tamponierten Nasennebenhöhle bzw. die Möglichkeit der intermittierenden Spülung der Nasennebenhöhle sichergestellt werden.

Die vorliegende Erfindung bietet hierfür umfassende Lösungsansätze auf der Grundlage mikro-dünnwandig ausgeführter Ballonhüllen aus wenig elastischem Polyurethan, die bereits bei der Herstellung zu residual bemessenen Ballonkörpern ausgeformt werden, die die Maße der zu versorgenden Kavitäten bzw. Ostien oder Kanäle bereits im Herstellungszustand, also ohne erforderliche elastische Aufdehnung überschreiten. In situ platziert, schlagen sich die residualen Hüllenanteile in Faltung in das Balloninnere ein, wodurch auch prominente, in den zu tamponierenden Raum reichende Strukturen mantelartig locker umschlagen werden und permanent wirkende Druckspitzen auf prominente Strukturen weitgehend vermieden werden können. Bei ausreichend residualer Bemaßung der mikro-dünnen Ballonhülle kann so die Oberfläche komplex geformter Binnenräume nahezu vollständig von der Tamponade flächig erfasst und inhomogene Kraftwirkung vermieden werden. Die radiale Bemaßung des tamponierenden Ballonkörpers kann daher großzügig ausgeführt werden um auch maximale Kavitätenvarianten mit dem beschriebenen Vorteil zu erfassen.

Die Anpassung der Länge des Tamponadekörpers bzw. seiner Segmente kann bei der Positionierung erfindungsgemäß auf verschiedene Weise erfolgen.

Das Ballonsegment, das innerhalb der Nasennebenhöhle platziert wird, kann als alternative Ausführung zum residualen Durchmesser einen relativ zur Kavität kleinen Durchmesser aufweisen und stattdessen residual lang ausgeführt werden. Bei der Platzierung wird es vom Operateur ähnlich wie ein Tamponadestreifen in die Kavität gestopft. Bei Befüllung staucht und faltet sich das überlange Segment in Art einer aufblasbaren Gaze in raumfüllender Weise in die Kavität. Überschüssige Ballonanteile gehen in Faltung, die Oberfläche der Kavität wird durch das entstehende Konvolut unter weitgehender Vermeidung lokaler Druckspitzen mit einer weitgehend homogenen Kraft belastet.

Die Längenausdehnung des Ballonsegmentes, welches sich bei Befüllung in die Nasenhaupthöhle entwickelt, kann durch ein die Entfaltung des Ballons begrenzendes, axial auf dem Schaftschlauch verschiebliches Hülsenelement eingestellt werden.

Zur Sicherung der Positionierung der Tamponade im Ostium wird diese in der bevorzugten Ausführung der Vorrichtung mit einer zirkulären Verjüngung bzw. Taille versehen, die in etwa dem Ostiendurchmesser entspricht, diesen aber bevorzugt überschreitet, so dass sich auch hier überschüssiges Hüllenmaterial radial einstülpt und eine homogene Druckwirkung auf die Wundflächen gewährleistet ist.

Durch das erfindungsgemäße Prinzip der mantelartigen, spannungslosen Faltung überschüssiger Hüllenwandung werden homogene Tamponadekräfte ermöglicht, die beispielsweise mittels eines Ballondruckmessers bei Anlage der Tamponade und vor allem auch im Tamponadeverlauf so eingestellt werden können, dass sie unterhalb der für die Schleimhautdurchblutung und damit unterhalb der für die Wundheilung erforderlichen Durchblutungsdrucke liegen. Der barometrisch gemessene Fülldruck entspricht bei dieser bevorzugten Ausführung der Tamponadesegmente in guter Näherung den jeweils trans-mural auf die Schleimhäute bzw. Wundflächen wirkenden Drucken.

Durch die guten Ankereigenschaften der Tamponade im Ostium sowie durch die Möglichkeit der Begrenzung der räumlichen Ausdehnung der Tamponade in die Haupthöhle hinein, kann auf eine den Tamponadekörper in seiner Position sichernden "Stopfung" der Haupthöhle verzichtet werden. Diese kann damit offenbleiben und so freie Nasenatmung gewährleisten. Das den Tamponadeballon tragende Schaftelement kann nach Anlage und Befüllung vom Operateur auf dem Niveau der äußeren Nasenöffnung getrimmt werden. Entsprechend kann die Befüllzuleitung, nach Einstellung der wirkenden Tamponadekraft, beispielsweise durch einen Clip verschlossen und ebenfalls auf Höhe der Nasenöffnung abgelängt werden. Die Tamponade kann somit ohne Beeinträchtigung der Nasenatmung und der Optik über längere Perioden vom Patienten getragen werden.

Für die Qualität der Wundheilung hat sich die insbesondere die Güte der Folienoberfläche als entscheidend herausgestellt. Je ebener die Folie, desto gleichmäßiger entwickeln sich die der Folie anliegenden, sich regenerierenden Gewebe. Unter starker axialer und radialer Streckung aus vorextrudiertem Schlauchmaterial geblasformte Ballonkörper aus Polyurethan (PUR) haben eine kaum zu übertreffende Oberflächengüte. Auch bei 10.000-facher Vergrößerung stellen sich die so ausgeformten PUR-Oberflächen immer noch tischeben dar.

Ferner entscheidet über den Heilungsverlauf, dass der Nasennebenhöhlenraum konstant belüftet wird bzw. dass durch das Lumen des Tamponadenschaftes hindurch der Sinus bei Bedarf auch gespült werden.

Weitere Merkmale, Einzelheiten, Vorteile und Wirkungen auf der Basis der Erfindung ergeben sich aus der folgenden Beschreibung bevorzugter Ausführungsformen der Erfindung sowie anhand der Zeichnung, worin das Funktions- und Gestaltungsprinzip der erfindungsgemäßen Tamponaden näher erläutert wird. Hierbei zeigt:
- Fig. 1: einen beispielhaften, frei entfalten Tamponadekörper;
- Fig. 2: beispielhaft einen der Fig. 1 entsprechenden Tamponadekörper in einer Stirnhöhle eingelegt und mit Fülldruck beaufschlagt;
- Fig. 2a: einen Querschnitt des Tamponadekörpers im Bereich des Ostiums;
- Fig. 2b: einen Querschnitt der eingelegten Tamponade im Bereich der Kavität der Stirnhöhle;
- Fig. 3: eine Ausführung der Tamponade mit residual verlängertem Nasennebenhöhlensegment in situ, mit gefalteter bzw. gestauchter raumfüllender Entwicklung des die Nasennebenhöhle ausfüllenden Ballonkörpers;
- Fig. 4: eine an Fig. 1 angelehnte Ausführungsform mit einem partiell zurückgestülpten, vorderen Ballonkörper;
- Fig. 5: ein Hülsenelement zur räumlichen Begrenzung des Nasenhaupthöhlensegmentes des Ballonkörpers;
- Fig. 5a: eine besondere Ablängung bzw. Markierung des Hülsenelementes;
- Fig. 6: einen befüllbaren Tamponadeschlauch mit perlenkettenartiger Struktur;
- Fig. 7: eine Ausführungsform der Blutungstamponade zur kompletten Tamponade der Nasenhaupthöhle;
- Fig. 7a: die entsprechende Tamponade in freier Entfaltung; sowie
- Fig. 8: eine beidseitige Tamponade zur postoperativen Versorgung einer Korrektur der Nasenscheidewand.

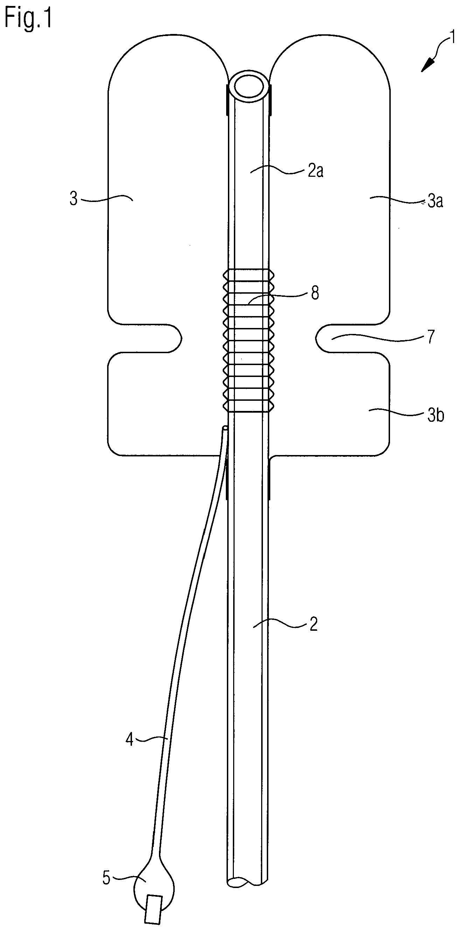

Fig. 1 stellt einen Tamponadekörper 1 dar, der einen einlumig ausgeführten Schaftschlauch 2 aufweist, welchem ein tailliert ausgeformter Ballonkörper 3 aufsitzt. Der Ballonkörper wird an seinem proximalen Ende durch eine kleinlumige Befüllzuleitung 4 mit einem Füllmedium beaufschlagt. Am proximalen Ende der Befüllzuleitung befindet sich ein Pilotballon 5 mit integriertem Einwegeventil.

Die gezeigte Tamponade ist baulich insbesondere für die Tamponade nach chirurgischen Zugangsweitungen zur Stirnhöhle ausgelegt. Der distale Anteil 3a des Ballons, der vor der Ballontaille 7 liegt, ist zylindrisch ausgeführt und weist beispielhaft einen Durchmesser von ca. 30 mm auf, bei einer Länge von ca. 50 mm. Der sich nach hinten an die Taille anschließende proximale Anteil 3b des Ballons hat beispielhaft einen Durchmesser von ca. 30 mm bei einer Länge von ca. 20 bis 30 mm. Im Bereich der Einschnürung 7 verjüngt sich der Ballon auf einen Durchmesser von bevorzugt ca. 5 bis 12 mm. Die Einschnürung oder Taille 7 wird bevorzugt mit möglichst kleinen Schulterradien versehen, so dass sich die Schultern der Taille im befüllten Zustand möglichst senkrecht darstellen, was die ankernde Wirkung der Taille im Ostium optimiert. Die Breite der Einschnürung beträgt bevorzugt ca. 3 bis 8 mm, besonders bevorzugt 4 bis 6 mm.

Die Ballonhülle selbst besteht in der bevorzugten Ausführung aus Polyurethan (PUR) der Härten Shore 80A bis 95A oder auch 55 bis 65D. Es kommen beispielsweise Materialien des Typs Elastollan der Fa. BASF oder Pellethane der Fa. Lubrizol in Frage. Die Wandungsstärken der Folie liegen im Bereich der zylindrischen Flächen der Ballonanteile 3a und 3b bei 5 bis 50 µm, bevorzugt jedoch bei 5 bis 15 µm. Im Bereich der Einschnürung sollte die Ballonwandung bevorzugt im Bereich von 15 bis 40 µm liegen. Der Ballonkörper 3 kann alternativ zu PUR auch aus PVC, LDPE, Pebax oder vergleichbaren Materialien mit ausreichender mechanischer Beständigkeit und dimensionaler Stabilität gefertigt werden. Bevorzugt sind Materialien, die sich vor allem bei Zugbelastung dimensional stabil und auch formstabil verhalten, um unerwünschte Schlupfeffekte zu vermeiden und eine zugstabile Ankerung der Tamponade im Ostium zu sichern. Stark volumendehnbare Materialien wie Silikon oder Latex, mit geringer Formstabilität und eine damit einhergehender hoher Schlupfneigung des Ballonkörpers werden im Rahmen der Erfindung nicht bevorzugt.

Der Schaftschlauch 2 wird in der bevorzugten Ausführung ebenfalls aus Polyurethan gefertigt. Er sollte möglichst weich sein, und im idealen Falle die spannungslose Flexibilität einer gekochten Makkaroni aufweisen, dabei aber, bei einem im Ballon wirkenden Fülldruck von 30 bis 60 mbar, das Schlauchlumen offenhalten. Der Schaft besteht bevorzugt aus Polyurethan der Härte Shore 60 A bis 90 A, besonders bevorzugt Shore 60 A bis 70 A. Kommt es zum passageren radialen Kollaps des Schlauchlumens, richten sich PUR-basierte Schaftschläuche bei nachlassender Kraft in der Regel prompt elastisch radial auf. Zur Vermeidung einer PUR-typischen, permanent wirkenden axialen elastischen Aufrichtung des Schaftschlauches, kann dieser partiell oder über seine gesamte Länge hinweg mit einer welligen Korrugation 8 versehen wird, die den sich elastisch axial ausrichtenden Schaft geschmeidig biegbar macht. Die wellige Korrugation kann zudem einen Lumenverschluß durch axiale Knickung bei Biegungen des Schlauches von bis zu 360 Grad vermeiden.

Der Schaftschlauch 2 weist vorzugsweise einen Durchmesser von außen 3 bis 4 mm und innen von 1,5 bis 2,5 mm auf. Das distale Ende 2a des Schaftes ist bevorzugt derart relativ zur distalen Ballonschulter platziert, dass es den vorderen Ballonradius im befüllten Zustand bei freier Entfaltung nicht überragt.

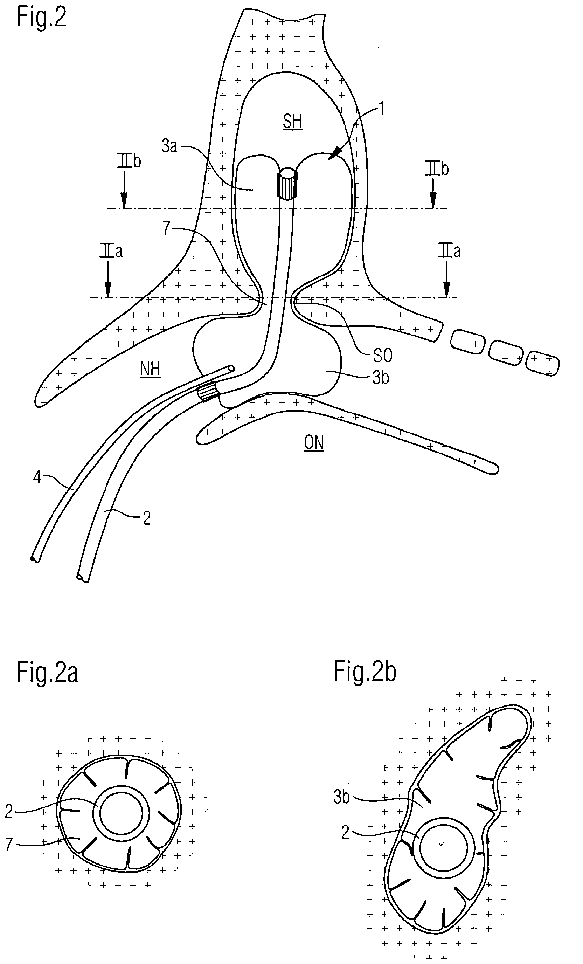

Fig. 2 zeigt einen erfindungsgemäßen Tamponadekörper 1 im Zugangskanal zur Stirnhöhle (Ductus naso-frontalis) platziert. Die tailliert eingeschnürte Portion 7 des Ballonkörpers wird im Ostium SO der Stirnhöhle platziert. Bei Befüllung des Ballons schmiegt sich die vordere Portion 3a des Ballonkörpers den Wandungen der Stirnhöhle an. Der hintere, proximale Anteil 3b des Ballons entwickelt sich bei Befüllung vor dem Ductus in den jeweilig angrenzenden, zur Entfaltung verfügbaren Raum der Nasenhöhle NH hinein. Der Entfaltungsraum wird durch die obere Nasenmuschel ON und das Nasenseptum definiert.

Vorteilhaft für die Tamponadeeigenschaften der Vorrichtung ist eine ausreichend residuale Bemaßung des Ballondurchmessers im vorderen Anteil 3a, die gewährleistet, dass sich die Ballonhülle dem jeweiligen Raum unter Ausbildung einer sich in das Innere der Ballons einstülpenden Faltung anschmiegt, wodurch eine optimal gleichmäßige Kraftübertragung auf die anliegende Schleimhaut ermöglicht wird. Bedingt durch die residuale Ausformung des Ballondurchmessers kann die sich durch den Ballon auf die anliegenden Strukturen übertragene Kraft in guter Näherung mit dem im Ballon wirkenden Fülldruck gleichgesetzt werden. Werden beispielsweise Fülldrucke von ca. 20 mbar im Ballon barometrisch durch einen entsprechenden Pumpregler eingestellt, kann davon ausgegangen werden, dass der auf das kapillare Gefäßbett wirkende Tamponadedruck ebenfalls bei ca. 20 mbar liegt. So kann, vom Anwender messbar und über den Verlauf der Tamponade hinweg, die trans-mural wirkenden Kräfte eingestellt und die kapillare Perfusion aufrechterhalten werden. Die besondere, für die Regulierung der Kraftwirkung auf das Gewebe entscheidende Faltung der extrem dünnwandigen, jeweils im Durchmesser residual bemessenen Ballonhülle wird in Abb. 2a und 2b schematisch verdeutlicht.

Fig. 2a stellt einen Schnitt durch den Kanal des Stirnhöhlenostiums SO dar. Durch die auch hier bevorzugte, im Durchmesser residuale Ausführung der Ballontaille 7 schmiegt sich das Segment jeder anzunehmenden Form und Dimension des Ostiums an, ohne zur allseitigen Tamponade des Ostiums in einen gespannten Zustand übergehen zu müssen. So kann insbesondere im Bereich der chirurgischen Weitung des Ostiums bzw. des Ductus eine von extrakorporal kontrollierbare und regelbare, perfusionskompatible Tamponadewirkung eingestellt werden.

Fig. 2b stellt die vorteilhafte Faltung der beschriebenen residualen Ballonhülle im Bereich der Stirnhöhle SH dar. Prominente Strukturen, welche in die Kavität hineinreichen, werden durch die Ballonhülle mantelartig umschlossen, ohne dabei Druckspitzen durch eine elastische Verformung der Ballonhülle zu erzeugen. Es stellt sich stattdessen eine weitgehend homogene Kraftwirkung der Tamponade auf die exponierten Flächen der Tamponade ein.

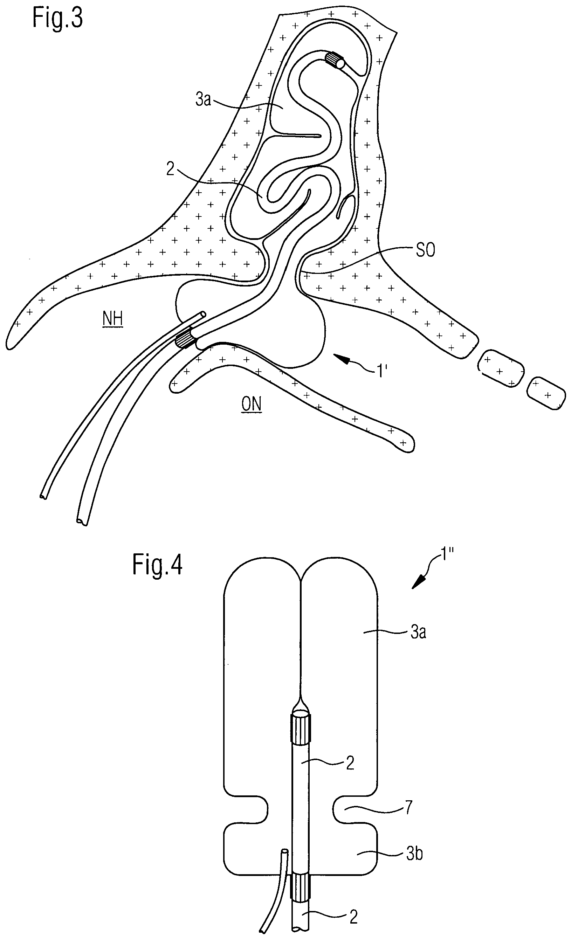

Fig. 3 zeigt als alternative Ausführungsform einer Tamponade 1' mit residual verlängertem Nasennebenhöhlensegment 3a, welches sich in der Stirnhöhle unter spontaner Faltung und Knickung über die Längsachse des Ballonkörpers raumfüllend, in konvolutartiger Weise hineinentwickelt. Für eine derartige Tamponade der Stirnhöhle wird die Länge des vorderen Ballonzylinders 3a auf 5 bis 10 cm, bevorzugt 5 bis 7 cm vergrößert. Der das vordere Ballonsegment tragende Schlauch 2 weist bei dieser Ausführung eine besonders geschmeidige, nicht elastisch federnde Biegbarkeit auf, die es ihm erlaubt, dem sich in der Kavität unter Füllung faltenden und knickenden Ballonkorpus möglichst spannungslos zu folgen. Das distale Ballonsegment 3a kann eine perlenkettenartig separierte Aneinanderreihung von sphärisch, zylindrisch oder diskoid ausgeformten Ballonsegmenten aufweisen. Hierbei liegt der Durchmesser der separierenden Einschnürungen bevorzugt um mindestens 50% unter dem Durchmesser der angrenzenden ausgeformten Segmente. Die Durchmesser der ausgeformten Segmente wiederum überschreiten in der bevorzugten Ausführung den Durchmesser des Ostiums bzw. Ductus um mindestens 50%.

Bei der Tamponade 1" nach Fig. 4 ist das vordere Ende des Ballonanteils 3a in den Ballonkorpus partiell zurückgestülpt und wird dort vom freien Ende des Schlauches 2 aufgenommen. Die Verbindungstelle zwischen Ballon 3 und Schlauch liegt hier bevorzugt mindestens 10 mm oberhalb der Balloneinschnürung 7. So kann bei Verwendung weniger geschmeidig biegsamerer Materialien des Trägerschlauches dennoch eine effizient raumfüllende Tamponade, wie in Fig. 3 beschrieben, erreicht bzw. ein eventueller traumatisierender Effekt eines zu starren, im Verlauf multipel geknickten Schaftes vermieden werden.

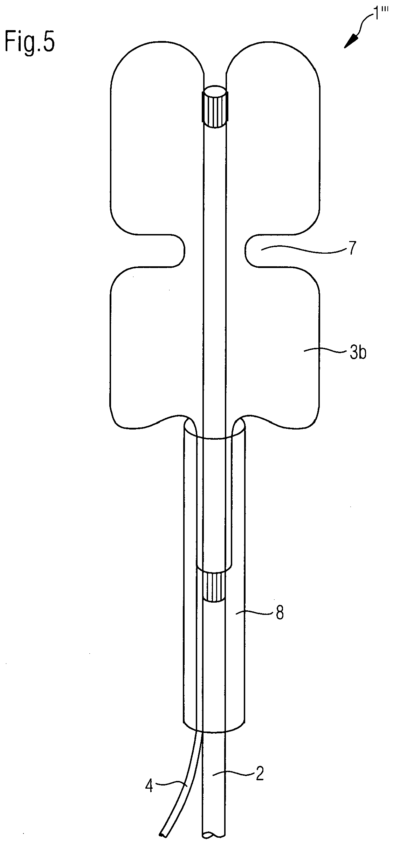

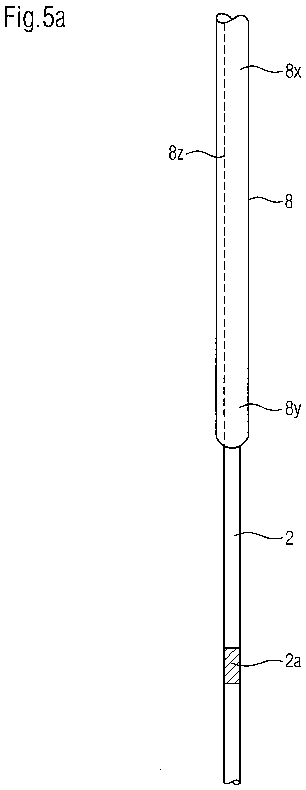

Fig. 5 zeigt eine Ausführungsform der Tamponade 1"', bei der das Ballonelement 3b als zylindrisch ausgeführtes Segment nach proximal verlängert wird. Es wird in seiner räumlichen Entfaltung durch ein übergestreiftes Schlauch- oder Hülsenelement 8 begrenzt, welches vom Operateur über dem Segment 3b verschoben bzw. zur Erreichung der gewünschten Ablängung platziert wird. Die axiale Länge des Segmentes 3b kann bei dieser Ausführung auf ca. 4 bis 8 cm, bevorzugt 4 bis 6 cm vergrößert werden. Die Option zur justierbaren Entfaltung der Tamponade im Bereich vor dem Ostium kann zum einen im individuellen Fall die Qualität der Ankerung der Tamponade optimieren und zum anderen eine Verlegung der Nasenhöhle vermeiden und somit die freie Nasenatmung ermöglichen.

In Fig. 5a wird eine besondere Ausführung des Hülsenelementes 8 sowie des Schaftschlauches 2 vorgestellt, die besondere Vorteile bei der intraoperativen Platzierung der Tamponade durch den Operateur erlaubt. Die Hülse 8 ist dabei derart gestaltet, dass der entleerte, nicht befüllte Tamponadekörper vollständig in der Hülse aufgenommen wird und sich innerhalb der Hülse bzw. relativ zur Hülse mit möglichst geringer Kraft möglichst in frei gleitender Weise verschieben lässt. Die Tamponade ist derart in die Hülse eingescheidet, dass das vordere, distale Ende des Tamponadekörpers mit dem distalen Ende 8x der Hülse bündig abschließt. Bei der Platzierung der Vorrichtung kann das so eingescheidete distale Ende der Tamponade mit der Spitze einer Pinzette gefasst und sicher in den Übergang der Nasenhaupt- in die Nasennebenhöhle eingelegt werden. Anschließend wird die Tamponade relativ zur vom Operateur manuell in dieser Position fixierten Hülse nach vorn, in die Nasennebenhöhle vorgeschoben. Erreicht dabei eine auf dem Schaftschlauch 2 aufgebrachte Markierung 2a das proximale Ende 8y der Hülse, ist der distale Anteil des Tamponadeballons 3a vollständig aus der Hülse 8 herausgeschoben und in der Nasennebenhöhle eingebracht. Die verjüngte Portion 7 des Tamponadekörpers liegt nun bündig mit dem distalen Ende 8x der Hülse. In dieser relativen Position von Nebenhöhlenostium, distalem Hülsenende 8x und Verjüngung 7 wird der distale Ballonanteil 3a dann mit einem ersten Füllvolumen beaufschlagt, wodurch er sich raumfüllend in die Nebenhöhle hineinentfaltet. Die Verjüngung 7 positioniert sich mit zunehmender Füllung im anatomischen Übergang, verankert die Vorrichtung so, und verhindert das Herausgleiten des befüllten distalen Ballonkörpers. Anschließend wird die Hülse 8 relativ zum so in fixierten Ballon zurückgezogen, so dass nun der proximale Ballonanteil 3b aus der Hülse 8 freigegeben wird. Der Tamponadeballon wird dann auf seinen endgültigen Füllzustand gebracht. Abschließend wird die Hülse vollständig aus der Nase herausgezogen und durch Aufreißen einer längsverlaufenden Perforation 8z geöffnet und von der Tamponade entfernt.

Ein erfindungsgemäßer, mit einem Medium befüllbarer Tamponadeschlauch wird vorzugsweise im nicht befüllten, evakuierten bzw. kollabierten Zustand nach Art eines Gazestreifens in eine durch ein Ostium abgegrenzte Kavität des Gesichtsschädels, quasi durch stopfende Führung mit einer Pinzette eingelegt. Bei der Befüllung des Tamponadeschlauches durch eine proximal angebrachte Befüllzuleitung entwickelt sich die Tamponade zu einer konvolutartigen, den jeweiligen Raum füllenden Struktur, die in der Lage ist, auch komplex geformte Kavitäten bei annähernd gleichmäßiger Kraftentwicklung auf deren Wandung in tamponierender Weise auszufüllen. Der Tamponadeschlauch benötigt kein tragendes Schaftelement. Die aufblasbare Stopftamponade kann nach der Anwendung evakuiert und schmerzlos entfernt werden.

Vorzugsweise besteht der Schlauchkorpus aus einem mikrodünnen PUR der Shore-Härte von 80A bis 90A und weist eine Wandstärke von 5 bis 50 Mikrometer auf; oder eine Shore-Härte bis 90A und eine Wandstärke von 5 bis 50 Mikrometer, bevorzugt 10 bis 12 Mikrometer auf.

Der Schlauchkorpus kann einen Durchmesser von 4 bis 20 mm aufweisen, vorzugsweise einen Durchmesser von 4 bis 12 mm, besonders bevorzugt einen Durchmesser von 5 bis 8 mm auf.

Der Schlauchkorpus kann eine residuale Länge aufweisen, welche die 3- bis 10-fache Länge der auszufüllenden Kavität haben kann.

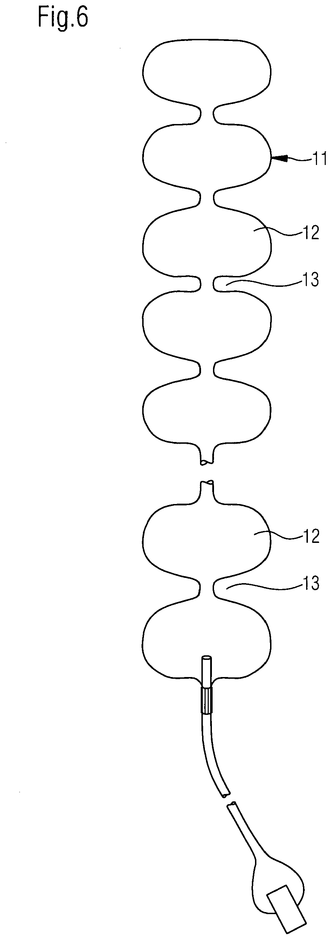

Fig. 6 zeigt einen entsprechenden Tamponadeschlauch 11 mit perlenkettenartiger Taillierung. Die segmentartigen Abschnürungen von sphärischen, zylindrischen oder diskoiden Segmenten 12 reduzieren die Wahrscheinlichkeit des Herausschlüpfens von Anteilen des sich innerhalb der Kavität aufknäulenden Tamponadenkonvolutes aus dem zur Kavität führenden Ostium. Im entstehenden Konvolut überlagern bzw. überkreuzen sich die Taillierungen 13 mehrfach. Das Konvolut wird so stabilisiert. Der Durchmesser der einzelnen Segmente 12 liegt dabei vorzugsweise um ca. 50% über dem Durchmesser des Ostiums. Die Durchmesser der Taillierungen 13 liegt vorzugsweise bei etwa der Hälfte des Durchmessers des Ostiums.

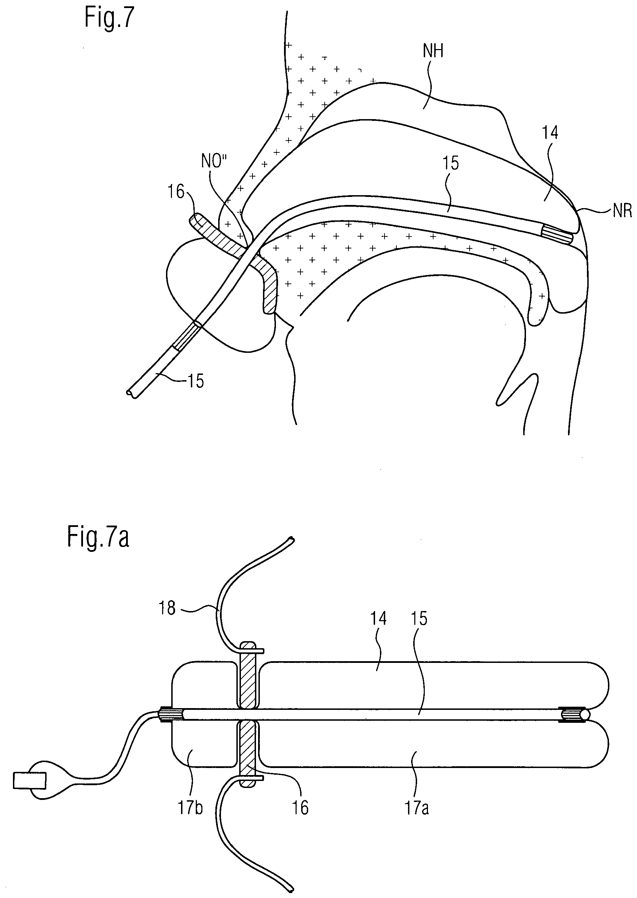

Fig. 7 zeigt eine Ausführung der Tamponade als Blutungstamponade 14 zur raumfüllenden Versorgung von Blutungen in der Nasenhaupthöhle NH. Die Längsausdehnung der Tamponade reicht von der vorderen Nasenöffnung NÖ bis in den Nasenrachen NR. im befüllten Zustand schließt sie so den Nasenhöhlenraum NH dicht ab und erlaubt die Anlage eines Blutungsstaudruckes, der zur Selbsttamponade von sickernden Blutungen in Bereichen der Nase führen kann, die durch die Tamponadewandung nicht direkt in eine blutstillende Kompression genommen werden können. Die Erfindung ermöglicht insbesondere die Selbsteinführung einer derartigen invasiven Tamponade die von der Nasenöffnung bis zur Hinterwand des Nasenrachens reicht. Die Erfindung schlägt einen im Wesentlichen zylindrischen Ballon vor, der im distalen Bereich eine Taillierung aufweist, die im Bereich des Übergangs der Nasenhöhle in Nasenrachen platziert wird. Das distale, im Nasenrachen platzierte Segment weist eine Länge von ca. 3 bis 4 cm auf und hat einen Durchmesser, der bevorzugt kleiner ist als der des Proximalen, intra-nasalen Ballonsegments. Der Ballonkorpus 17 sitzt einem Schaftschlauch 15 von etwa 3,5 bis 5 mm Außendurchmesser auf. Der Schaft weist eine ausreichende axiale Stabilität auf, das die Tamponade im evakuierten Zustand ohne weitere Hilfsmittel vom Patienten sicher geführt und nur mit minimaler Irritation in den unteren Nasengang eingeführt werden kann.

Die Tamponade weist im vorderen Bereich vorzugsweise ein frei verschiebliches, diskoides Element 16 mit einer durchgehenden Ausnehmung auf. Nach Einführung der Tamponade kann dieses scheibenartige Element 16 mit seiner Ausnehmung von proximal nach distal über den Tamponadekörper 14 gestreift werden, bis es der Nasenöffnung anliegt. Die verschiebliche Disk 16 sichert so die Position der Tamponade und fixiert diese in der gewünschten Einführtiefe. Dabei besteht das diskoide Element 16 aus einem harten Material und bildet somit eine harte Struktur im Bereich des Nasenostiums, an welcher der Ballon des Tamponadekörpers 14 sich verjüngt und dadurch in dem Bereich seines Übergangs zwischen distalem Segment (3a) des Ballons (3) und proximalem Segment (3b) des Ballons (3) an dem scheibenförmigen Element 16 im Bereich des Ostiums verankert wird.

Bei der anschließenden Befüllung entfaltet sich bevorzugt zuerst das distale Ballonsegment, was durch die Zuleitung des Füllmediums Öffnungen im distalen Drittel der Tamponade erreicht wird. Das durch die Disk 16 begrenzte proximale Ballonsegment füllt sich dann sukzessive durch Überstrom von Medium, welches durch den vorzugsweisen engen, unter elastischer Spannung anliegenden Spalt zwischen Disk und Schaft in dieses abfließt. Es bilden sich somit also ein proximales Segment vor der Disk bzw. vor der Nasenöffnung aus, welches zur Stabilisierung der Position bzw. der Einführtiefe der Tamponade beiträgt. Die Disk 16 kann mit einem Halteband 18 versehen sein, welches in Art einer Sauerstoffbrille über die Rückseite der Ohren geschlungen und unter dem Kinn durch ein Hülsenelement fixiert wird.

Fig. 7a zeigt die Tamponade 14 in freier Entfaltung. Das scheibenartige Element 16 kann auf dem Schaftschlauch unter elastischer Spannung frei verschoben werden. Es begrenzt so die Einführtiefe bzw. räumliche Ausdehnung des distalen Ballonsegmentes 17a in die Nasenhöhle bzw. in den Nasenrachenraum. Die Tamponade wird bevorzugt durch ein Einwegeventil über eine kurze Schlauchzuleitung befüllt.

In Abwandlung von der in Fig. 7 beschriebenen Ausführungsform können die vorliegenden Blutungstamponaden Längen aufweisen, die lediglich bis in das vordere, mittlere oder hintere Nasendrittel reichen. Es sind auch Tamponaden vorstellbar, die tamponierende Segmente nur in einzelnen Abschnitten der Nasenhöhle ausbilden, beispielsweise das vordere Drittel nicht erreichen, aber im mittleren und hinteren Drittel der Nase wirken. Die distalen Anteile des tamponierenden Ballons können dann einen entsprechend tamponade-unwirksamen Durchmesser aufweisen.

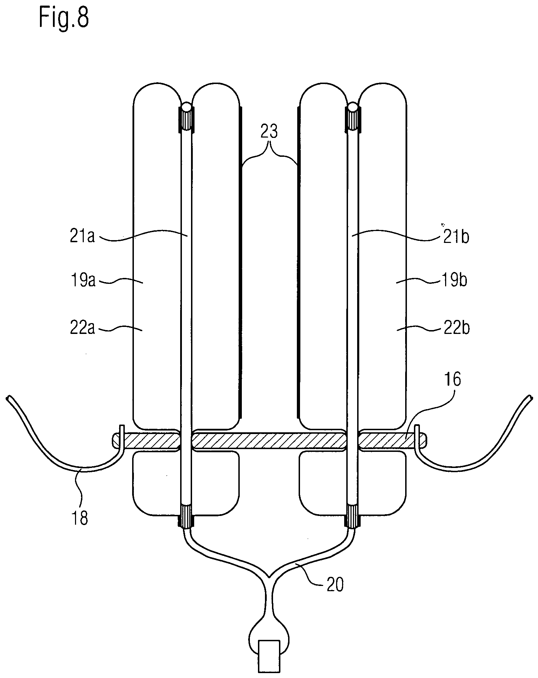

Fig. 8 zeigt im Rahmen der Erfindung zwei funktionell gekoppelte Tamponadekörper 19a, 19b, die für die Tamponade der Nasenhaupthöhle nach Korrekturen der Nasenscheidewand eingesetzt werden können. Die beiden Tamponadenschenkel 19a und 19b sind am vorderen Ende durch eine y-artige Struktur 20 verbunden, die dem Nasensteg quasi reitend aufsitzt. Über das innere Lumen des y-artigen Verbinders werden beide Ballonanteile gemeinsam befüllt und sind diese miteinander frei kommunizierend verbunden.

Die beiden Tamponadekörper 19a, 19b können außer über einen gemeinsamen Befüllungsschlauch 20 auch über ein gemeinsames, scheibenartiges Element 16 miteinander gekoppelt sein, welches in diesem Falle zwei Aufnehmungen oder Ostien zur Hindurchführung je eines Tamponadekörpers 19a, 19b aufweist.

Die freie Kommunikation der beiden Ballonschenkel sowie die mikro-dünne, residual bemessene Ausführung der verwendeten Ballonhüllen stellen das operativ korrigierte Nasenseptum in einer präzisen mittigen Position ein. Zusätzlich können die beiden Ballone 22a, 22b der Tamponadekörper 19a, 19b stabilisierende, plättchenartig starre Körper 23 zur beidseitigen Anlage und Schienung des frakturierten, noch instabilen knöchernen Septums aufweisen.

Sämtliche erfindungsgemäß beschriebenen Tamponaden können optional unter Verwendung eines führenden Mandrins eingeführt werden, der im Anschluss an die Anlage aus der Tamponade entfernt wird.

Die Befüllung der Tamponadekörper erfolgt bevorzugt mit Luft als Medium.

Von besonderem Vorteil bei Anwendung der erfindungsgemäßen Technologie ist ferner die bei der beschriebenen Ausführung in mikro-dünnem Polyurethan mögliche optische Transparenz des Ballon-Materials, die es dem Operateur erlaubt, durch die Ballonhülle optisch weitgehend unbeeinträchtigt hindurchzusehen und den Zustand der Wunde bzw. des Gewebes, welches von der Tamponade erfasst ist, direkt zu inspizieren. Bei zu hohem Tamponadedruck kann so z.B. eine Blassfärbung des Gewebes gesehen und erkannt werden. Der Operateur kann dann durch Reduktion des Fülldrucks in der Tamponade, bei Verwendung von Luft als Medium, von außen gut manometrisch einstellbar, rosig durchblutete Gewebe einstellen, die den Verlauf der Wundheilung positiv unterstützen.

Ein weiterer, wichtiger Gesichtspunkt ist eine möglichst glatte Beschaffenheit der Außenseite des Ballons eines erfindungsgemäßen Tamponadekörpers. Eine mittlere Rest-Rauheit sollte kleiner sein als 5 µm, beispielsweise kleiner als 2 µm, vorzugsweise kleiner als 1 µm, insbesondere kleiner als 0,5 µm.

Der erfindungsgemäße Tamponadekörper ist für Kavitäten im Bereich des Nasentraktes konzipiert, wo der auszufüllende Raum naturgemäß eine gewisse Starre seiner Wandung sowie eine innere räumliche Komplexität aufweist. Durch das beschriebene Prinzip der residualen Dimensionierung können diese Räume bei kleiner, flächig homogen wirkender Druckkraft perfusionskompatibel tamponiert werden.

## Patentansprüche

1. Nasenhöhlen- oder Nasennebenhöhlentamponade (1;1';1";1"';11;14;19a,19b) zur krafthomogen wirkenden Tamponade einer Kavität des Nasentraktes im Bereich eines zu der Kavität führenden Ostiums, umfassend einen dünnwandigen Ballon (3) aus einem weichfolienartigen, nur gering dehnbaren, sich geschmeidig faltenden Polyurethan-Material mit einer Wandstärke in dem Bereich von 5 bis 50 µm, welcher ein distales Segment (3a) aufweist, sowie ein proximales Segment (3b) und ein das distale Segment (3a) des Ballons (3) mit dem proximalen Segment (3b) des Ballons (3) verbindendes, transostiales Segment, **dadurch gekennzeichnet, dass** der Ballon (3) bereits bei der Herstellung vollständig auf sein erforderliches Tamponademaß oder darüber hinaus ausgeformt ist und dabei im Bereich des Übergangs vom distalen, innerhalb der zu tamponierenden Kavität gelegenen Ballonsegment (3a) in den Bereich des proximalen, vor der Kavität gelegenen Ballonsegmentes (3b) mit einer präformierten, taillen- oder sanduhrförmigen Verjüngung (7,13) oder Einschnürung versehen ist, und der in in situ eingesetztem Zustand des Ballons (3) in einen befüllten Zustand aufdehnbar ist, in welchem sich das distale Segment (3a) des Ballons (3) innerhalb der zu tamponierenden Kavität befindet und diese, in der Regel irregulär geformte Kavität bei möglichst gleichförmiger Kraftentwicklung auf die dem Ballon (3) exponierten Strukturen ausfüllt, während sich das proximale Segment (3b) des Ballons (3) vor der Kavität befindet und sich das transostiale Segment des Ballons (3) durch das Ostium hindurch erstreckt und die Nasenhöhlen- oder Nasennebenhöhlentamponade (1;1';1";1'";11;14;19a,19b) im Bereich des Übergangs an dem Ostium verankert und in ihrer Position verlässlich sichert.

2. Nasenhöhlen- oder Nasennebenhöhlentamponade (1;1';1";1"';11;14;19a,19b) nach Anspruch 1, **dadurch gekennzeichnet, dass** der Ballon (3) derart residual ausgeformt ist, daass er ohne erforderliche elastische Aufdehnung
a) der Oberfläche der Kavität und/oder des Ostiums unter Ausprägung multipler, weitgehend spannungsloser Faltung der Ballonhülle anliegt, und/oder
b) der Oberfläche der Kavität unter weitgehender Vermeidung von verbleibenden Resträumen anliegt.

3. Nasenhöhlen- oder Nasennebenhöhlentamponade (1;1';1";1"';11;14;19a,19b) nach Anspruch 1 oder 2, **dadurch gekennzeichnet, dass** die Hülle des Ballons (3) im Bereich des Ostiums spannungslos ist und einer dort angeordneten, festen, d.h. nicht dehnbaren Struktur ohne spannungsbedingten Druck anliegt, so dass der auf die Oberfläche des Ostiums ausgeübte Flächendruck dem auf die Oberfläche der Kavität ausgeübten Flächendruck entspricht.

4. Nasenhöhlen- oder Nasennebenhöhlentamponade (1;1';1";1''';11;14;19a,19b) nach einem der vorausgehenden Ansprüche, **dadurch gekennzeichnet, dass** das Polyurethan-Material des Ballons (3) in dem Durometerbereich Shore 80A bis 95A liegt.

5. Nasenhöhlen- oder Nasennebenhöhlentamponade (1;1';1";1"';11;14;19a,19b) nach einem der vorausgehenden Ansprüche, **dadurch gekennzeichnet, dass** der Ballon (3), insbesondere im Bereich seines größten Durchmessers, eine Wandungsstärke von 5 bis 25 µm aufweist, und/oder eine Wandungsstärke von 10 bis 20 µm.

6. Nasenhöhlen- oder Nasennebenhöhlentamponade (1;1';1";1"';11;14;19a,19b) nach einem der vorausgehenden Ansprüche, **dadurch gekennzeichnet, dass** der Ballon (3) aus einem optisch transparenten Material besteht, welches die visuelle Inspektion des der Nasenhöhlen- oder Nasennebenhöhlentamponade (1;14;19a,19b) anliegenden Gewebes ermöglicht.

7. Nasenhöhlen- oder Nasennebenhöhlentamponade (1;1';1";1"';11;14;19a,19b) nach einem der vorhergehenden Ansprüche, **dadurch gekennzeichnet, dass** der Durchmesser der vorzugsweise präformierten, taillen- oder sanduhrförmigen Verjüngung (7,13) oder Einschnürung um wenigstens 50% kleiner ist als der Durchmesser des distal angrenzenden Ballonsegments.

8. Nasenhöhlen- oder Nasennebenhöhlentamponade (1;1';1";1''';11;14;19a,19b) nach einem der vorausgehenden Ansprüche, **dadurch gekennzeichnet, dass** das distale Segment (3a) des Ballons (3)
a) derart residual verlängert ist, dass es sich bei Platzierung in der auszufüllenden Kavität sowie unter Befüllung in Form eines gefalteten und/oder gewundenen, raumfüllenden Konvolutes entwickelt, und/oder
b) eine perlenkettenartige Aneinanderreihung von sphärisch, zylindrisch oder diskoid ausgeformten, durch Verjüngungen (13) oder Einschnürungen voneinander separierten Ballonsegmenten (12) aufweist, und/oder
c) die zwei- bis zehnfache, bevorzugt die zwei- bis fünffache Länge der zu tamponierenden Kavität aufweist.

9. Nasenhöhlen- oder Nasennebenhöhlentamponade (1;1';1";1"';11;14;19a,19b) nach einem der vorhergehenden Ansprüche, **dadurch gekennzeichnet, dass** der Ballon (3) an seinem distalen Ende keine Öffnung aufweist, oder dass eine dortige Öffnung blind verschlossen ist.

10. Nasenhöhlen- oder Nasennebenhöhlentamponade (1;1';1";1'";11;14;19a,19b) nach einem der vorhergehenden Ansprüche, **dadurch gekennzeichnet, dass** der Ballon (3) einem durchgehenden Trägerschlauch (2) aufsitzt, vorzugsweise wobei der Trägerschlauch (2) an seinem distalen Ende keine Öffnung aufweist, oder dass eine dortige Öffnung blind verschlossen ist.

11. Nasenhöhlen- oder Nasennebenhöhlentamponade (1;1';1";1"';11;14;19a,19b) nach Anspruch 9 oder 10, **dadurch gekennzeichnet, dass** lediglich das proximale Ende des Ballons (3) auf dem Trägerschlauch (2) aufgebracht ist.

12. Nasenhöhlen- oder Nasennebenhöhlentamponade (1;1';1";1'";11;14;19a,19b) nach einem der Ansprüche 9 bis 11, **dadurch gekennzeichnet, dass** der Trägerschlauch (2) bzw. Befüllschlauch in das distale, die Nasennebenhöhle ausfüllende Segment (3a) des Ballons (3) hineinreicht und dort an seinem distalen Ende mit dem distalen Ende des Ballons (3) dicht schließend verbunden ist.

13. Nasenhöhlen- oder Nasennebenhöhlentamponade (1;1';1";1''';11;14;19a,19b) nach einem der Ansprüche 9 bis 12, **dadurch gekennzeichnet, dass** der Trägerschlauch (2)
a) folienartig dünn ausgebildet ist, und/oder
b) elastisch verformbar ist, und/oder
c) eine Wandstärke von 0,1 bis 0,3 mm aufweist, und/oder
d) zur Verbesserung seiner Aufrichtungseigenschaften mit einer welligen Korrugation ausgeformt ist.

14. Nasenhöhlen- oder Nasennebenhöhlentamponade (1;1';1";1'";11;14;19a,19b) nach einem der vorausgehenden Ansprüche, **dadurch gekennzeichnet, dass** die Ausbildung des proximalen Segmentes (3b) des Ballons (3) bzw. dessen Längenausdehnung durch ein über den Trägerschlauch (2) und den kollabierten Ballon (3) geschobenes Hülsenelement (8) begrenzt werden kann.

15. Nasenhöhlen- oder Nasennebenhöhlentamponade (1;1';1";1'";11;14;19a,19b) nach einem der vorausgehenden Ansprüche, **dadurch gekennzeichnet, dass** zur gleichzeitigen Tamponade von Kavitäten in der linken und rechten Hälfte des Gesichtsschädels zwischen den beiden Ballonen (22a,22b) eine freie Verbindung hergestellt wird, beispielsweise durch eine gemeinsame Fixierungsplatte (16), welche die dazwischen liegenden anatomischen Strukturen bei beidseitig homogener Kraftwirkung zwischen sich in dieser Position fixiert, vorzugsweise wobei ein oder zwei, zur gleichzeitigen Tamponade von Kavitäten in der linken und rechten Hälfte des Gesichtsschädels vorgesehene Ballone (22a,22b) eine oder je eine Schiene (23) aus einem festen Material tragen, wobei zwei derartige Schienen (23) vorzugsweise einander zugewandt sind.

## Claims

1. Tamponade (1;1';1";1''';11;14;19a,19b) for nasal cavities or sinus cavities, for a force-homogenous tamponade of a cavity of the nasal tract in the area of an ostium leading to the cavity, comprising a thin-walled balloon (3) made of a soft-foil-like, only less stretchable, smoothly foldable polyurethane material with a wall thickness in a range of 5 to 50 µm, which has a distal segment (3a), as well as a proximal segment (3b) and a trans-ostial segment conjoining the distal segment (3a) of the balloon (3) with the proximal segment (3b) of the balloon (3), **characterized in that** the balloon (3) is shaped into ist required tamponade extent, or beyond, already at ist manufacturing, and, at that time, is provided with a preformed, waist-like or hourglass-shaped tapering (7,13) or neck in the area of the transition from the distal balloon segment (3a) located inside the cavity to be tamponated into the area of the proximal balloon segment (3b) located in front of the cavity, and which, upon in situ insertion of the balloon (3), is expandable into a filled shape, wherein the distal segment (3a) of the balloon (3) is located inside the cavity to be tamponated and fills this normally irregularly formed cavity with a force incurrence on the structures exposed to the balloon as uniformly as possible, while the proximal segment (3b) of the balloon (3) is located in front of the cavity and the trans-ostial segment of the balloon (3) extends through the ostium and anchors the tamponade (1;1';1";1'";11;14;19a,19b) for nasal cavities or sinus cavities in the area of the transition at the ostium and reliably secures it in its position.

2. Tamponade (1;1';1";1''';11;14;19a,19b) for nasal cavities or sinus cavities according to claim 1, **characterized in that** the balloon (3) is shaped residually in such a way that it, without a necessary elastic expansion,
a) abuts the surface of the cavity and/or of the ostium by manifestation of multiple, largely tensionless foldings of the balloon envelope, and/or
b) abuts the surface of the cavity under avoidance of remaining residual spaces.

3. Tamponade (1;1';1";1"';11;14;19a,19b) for nasal cavities or sinus cavities according to claim 1 or 2, **characterized in that** the envelope of the ballon (3) is tensionless in the area of the ostium and abuts a rigid, i.e. non-expandable structure there without any pressure caused by tension, so that the surface pressure exerted onto the surface of the ostium corresponds to the surface pressure exerted onto the surface of the cavity.

4. Tamponade (1;1';1";1"';11;14;19a,19b) for nasal cavities or sinus cavities according to one of the preceding claims, **characterized in that** the polyurethane material of the ballon (3) is within a durometer range of Shore 80A to 95A.

5. Tamponade (1;1';1";1'";11;14;19a,19b) for nasal cavities or sinus cavities according to one of the preceding claims, **characterized in that** the balloon (3), especially in the area of its greatest diameter, has a wall thickness from 5 to 25 µm, and/or a wall thickness from 10 to 20 µm.

6. Tamponade (1;1';1";1'";11;14;19a,19b) for nasal cavities or sinus cavities according to one of the preceding claims, **characterized in that** the balloon (3) is made of an optically transparent material, which enables the visual inspection of the tissue abutting the tamponade (1;14;19a,19b) for nasal cavities or sinus cavities.

7. Tamponade (1;1';1";1'";11;14;19a,19b) for nasal cavities or sinus cavities according to one of the preceding claims, **characterized in that** the diameter of the preferably preformed, waist-like or hourglass-shaped tapering (7,13) or neck is at least 50 % smaller than the diameter of the balloon segment distally adjacent thereto.

8. Tamponade (1;1';1";1'";11;14;19a,19b) for nasal cavities or sinus cavities according to one of the preceding claims, **characterized in that** the distal segment (3a) of the balloon (3)
a) is residually lengthened in such a way that it develops in the form of a folded and/or wreathed, space-filling convoluted tubule upon placement inside the cavity to be filled as well as under inflation, and/or
b) exhibits a chaplet-shaped concatenation of spherically, cylindrically or discoidally formed balloon segments (12) separated from each other by taperings (3) or necks, and/or
c) exhibits a length double to decuple, preferably double to quintuple the length of the cavity to be tamponated.

9. Tamponade (1;1';1";1'";11;14;19a,19b) for nasal cavities or sinus cavities according to one of the preceding claims, **characterized in that** the balloon (3) does not exhibit any opening at its distal end, or that an opening there comprises a blank closure.

10. Tamponade (1;1';1";1"';11;14;19a,19b) for nasal cavities or sinus cavities according to one of the preceding claims, **characterized in that** the balloon (3) is mounted on a full-length supporting tube (2), preferably wherein the supporting tube (2) does not exhibit any opening at its distal end, or wherein an opening there comprises a blank closure.

11. Tamponade (1;1';1";1"';11;14;19a,19b) for nasal cavities or sinus cavities according to claim 9 or 10, **characterized in that** the proximal end of the balloon (3) is merely applied to the supporting tube (2).

12. Tamponade (1;1';1";1'";11;14;19a,19b) for nasal cavities or sinus cavities according to one of claims 9 to 11, **characterized in that** the supporting tube (2) or, respectively, the filling tube extends into the distal balloon segment (3a) filling the sinus cavity, and is tightly connected there with its distal end to the distal end of the balloon (3).

13. Tamponade (1;1';1";1'";11;14;19a,19b) for nasal cavities or sinus cavities according to one of claims 9 to 12, **characterized in that** the supporting tube (2)
a) is formed as thin as a foil, and/or
b) is resiliently malleable, and/or
c) exhibits a wall thickness from 0.1 to 0.3 mm, and/or
d) is formed with a wavy corrugation in order to improve its erection properties.

14. Tamponade (1;1';1";1"';11;14;19a,19b) for nasal cavities or sinus cavities according to one of the preceding claims, **characterized in that** the formation of the proximal segment (3b) of the balloon (3) or, respectively, the longitudinal extention thereof, can be limited by a sleeve element (8) shoved upon the supporting tube (2) and the collapsed balloon (3).

15. Tamponade (1;1';1";1"';11;14;19a,19b) for nasal cavities or sinus cavities according to one of the preceding claims, **characterized in that** a free connection between two balloons (22a,22b) is established for a simultaneous tamponade of cavities in the left and right half of the facial skull, for example by a common fixing plate (16) fixing the intermediate anatomical structures under bilateral homogenious force exertion, preferably wherein one or two balloons (22a,22b) provided for a simultaneous tamponade of cavities in the left and right half of the facial skull support a rail (23), or one rail (23) each, made of a rigid material, wherein two such reails (23) are preferably facing each other.

## Revendications

1. Tamponnement des cavités nasales ou des sinus (1 ; 1' ; 1" ; 1"' ; 11 ; 14 ; 19a, 19b) permettant de réaliser un tamponnement, agissant avec une force homogène, d'une cavité de la voie nasale dans la région d'un ostium menant jusqu'à la cavité, comportant un ballonnet à paroi mince (3) constitué d'un matériau de polyuréthane de type feuille souple, seulement légèrement extensible, se pliant de manière souple d'une épaisseur de paroi située dans la plage de 5 à 50 µm, lequel présente un segment distal (3a) ainsi qu'un segment proximal (3b) et un segment transostial reliant le segment distal (3a) du ballonnet (3) au segment proximal (3b) du ballonnet (3), **caractérisé en ce que** le ballonnet (3) est entièrement formé à sa dimension de tamponnement nécessaire ou au-delà de celle-ci dès la fabrication et comportant, dans la région de transition du segment de ballonnet (3a) situé de manière distale à l'intérieur de la cavité à tamponner dans la région du segment de ballonnet proximal (3b) situé en amont de la cavité, un rétrécissement préformé, de forme cintrée ou en sablier (7, 13) ou constriction, et qui est dilatable dans un état rempli à un état d'utilisation in situ du ballonnet (3), dans lequel le segment distal (3a) du ballonnet (3) se trouve à l'intérieur de la cavité à tamponner et remplit cette cavité généralement de forme irrégulière à génération d'une force aussi uniforme que possible sur les structures exposées au ballonnet (3), tandis que le segment proximal (3b) du ballonnet (3) se trouve en amont de la cavité et le segment transostial du ballonnet (3) s'étend à travers l'ostium et le tamponnement des cavités nasales ou des sinus (1 ; 1' ; 1" ; 1"' ; 11 ; 14 ; 19a, 19b) s'ancre dans la région de la transition à l'ostium et le bloque de manière fiable dans sa position.

2. Tamponnement des cavités nasales ou des sinus (1 ; 1' ; 1" ; 1'" ; 11 ; 14 ; 19a, 19b) selon la revendication 1, **caractérisé en ce que** le ballonnet (3) est formé de manière résiduelle de telle sorte qu'il s'applique sans dilatation élastique nécessaire
a) à la surface de la cavité et/ou de l'ostium moyennant la formation de pliagemultiple, en grande partie sans tension de l'enveloppe du ballonnet, et/ou
b) à la surface de la cavité en évitant dans une large mesure des espaces résiduels restants.

3. Tamponnement des cavités nasales ou des sinus (1; 1' ; 1" ; 1'" ; 11 ; 14 ; 19a, 19b) selon la revendication 1 ou 2, **caractérisé en ce que** l'enveloppe du ballonnet (3) dans la région de l'ostium est sans tension et qu'elle est appliquée à une structure qui y est disposée de manière fixe, c'est-à-dire une structure non dilatable sans pression liée à une tension, de sorte que la pression superficielle exercée à la surface de l'ostium correspond à la pression superficielle exercée sur la surface de la cavité.

4. Tamponnement des cavités nasales ou des sinus (1 ; 1' ; 1" ; 1''' ; 11 ; 14 ; 19a, 19b) selon l'une des revendications précédentes, **caractérisé en ce que** le matériau de polyuréthane du ballonnet (3) est compris dans la plage de duromètre Shore entre 80 A et 95 A.

5. Tamponnement des cavités nasales ou des sinus (1 ; 1' ; 1" ; 1"'; 11 ; 14; 19a, 19b) selon l'une des revendications précédentes, **caractérisé en ce que** le ballonnet (3), en particulier dans la zone de son plus grand diamètre, présente une épaisseur de paroi de 5 à 25 µm, et/ou une épaisseur de paroi de 10 à 20 µm.

6. Tamponnement des cavités nasales ou des sinus (1 ; 1' ; 1" ; 1"' ; 11 ; 14 ; 19a, 19b) selon l'une des revendications précédentes, caractérisé en ce quele ballonnet (3) est constitué d'un matériau optiquement transparent, lequel permet l'inspection visuelle du tissu adjacent au tamponnement des cavités nasales ou des sinus (1 ; 14 ; 19a, 19b).

7. Tamponnement des cavités nasales ou des sinus (1 ; 1' ; 1" ; 1"' ; 11 ; 14 ; 19a, 19b) selon l'une des revendications précédentes, **caractérisé en ce que** le diamètre du rétrécissement de préférence préformé, de forme cintrée ou en sablier (7, 13) ou constriction est inférieur d'au moins 50% au diamètre du segment de ballonnet distalement contigu.

8. Tamponnement des cavités nasales ou des sinus (1 ; 1' ; 1" ; 1'''; 11 ; 14 ; 19a, 19b) selon l'une des revendications précédentes, **caractérisé en ce que** le segment distal (3a) du ballonnet (3)
a) est allongé de manière résiduelle de telle sorte qu'il se développe lors du placement dans la cavité à remplir ainsi que sous remplissagesousforme d'un ensemble plié et/ou tordu remplissant l'espace, et/ou
b) présenteune juxtaposition en forme de chaîne de perles de segmentsde ballonnet (12) formés de manière sphérique, cylindrique oudiscoïdale, séparéslesuns des autres par des rétrécissements (13) ou constrictions, et/ou.
c) présente deux à dixfois, de préférence deux à cinq fois la longueur de la cavité à tamponner.

9. Tamponnement des cavités nasales ou des sinus (1 ; 1' ; 1" ; 1'" ; 11 ; 14 ; 19a, 19b) selon l'une des revendications précédentes, **caractérisé en ce que** le ballonnet (3) ne comporte pas d'ouverture au niveau de son extrémité distale, ou **en ce qu'**une ouverture y est obturée.

10. Tamponnement des cavités nasales ou des sinus (1 ; 1' ; 1" ; 1'" ; 11 ; 14 ; 19a, 19b) selon l'une des revendications précédentes, **caractérisé en ce que** le ballonnet (3) repose sur un tuyau flexible porteur (2) continu, de préférence **en ce que** le tuyau flexible porteur (2) ne présente pas d'ouverture au niveau de son extrémité distale, ou **en ce qu'**une ouverture y est obturée.

11. Tamponnement des cavités nasales ou des sinus (1 ; 1' ; 1" ; 1'" ; 11 ; 14 ; 19a, 19b) selon la revendication 9 ou 10, **caractérisé en ce qu'**uniquement l'extrémité proximale du ballonnet (3) est appliquée sur le tuyau flexible porteur (2).

12. Tamponnement des cavités nasales ou des sinus (1 ; 1' ; 1" ; 1'" ; 11 ; 14 ; 19a, 19b) selon l'une des revendications 9 à 11, **caractérisé en ce que** le tuyau flexible porteur (2) ou tuyau de remplissage entre dans le segment distal (3a) du ballonnet (3) remplissant le sinus et **en ce qu'**il y est raccordé hermétiquementau niveau de son extrémité distale à l'extrémité distale du ballonnet (3).

13. Tamponnement des cavités nasales ou des sinus (1 ; 1' ; 1" ; 1''' ; 11 ; 14 ; 19a, 19b) selon l'une des revendications 9 à 12, **caractérisé en ce que** le tuyau flexible porteur (2)
a) est de type feuille mince, et/ou
b) est élastiquement déformable, et/ou
c) présente une épaisseur de paroi comprise entre 0,1 et 0,3 mm, et/ou
d) est formé afin d'améliorer ses caractéristiques de redressement avec une ondulation ondulée.

14. Tamponnement des cavités nasales ou des sinus (1 ; 1' ; 1" ; 1'" ; 11 ; 14 ; 19a, 19b) selon l'une des revendications précédentes, **caractérisé en ce que** la formation du segment proximal (3b) du ballonnet (3) ou de son extension longitudinale peut être limitée par un élément de manchon (8) glissé sur le tuyau flexible porteur (2) et le ballonnet (3) affaissé.

15. Tamponnement des cavités nasales ou des sinus (1 ; 1' ; 1" ; 1'" ; 11 ; 14 ; 19a, 19b) selon l'une des revendications précédentes, **caractérisé en ce qu'**une liaison libre est réalisée pour le tamponnement simultané de cavités dans la moitié gauche et droite du massif facial entre les deux ballonnets (22a, 22b), par exemple par une plaqué de fixation (16) commune, laquelle fixe dans cette position les structures anatomiques intermédiaires à exercice d'une force homogène des deux côtés entre elles, de préférence **en ce qu'**un ou deux ballonnets (22a, 22b) prévus pour le tamponnement simultané de cavités dans la moitié gauche et droite du massif facial portent un ou chacun un rail (23) constitué d'un matériau solide, **en ce que** deux tels rails (23) sont de préférence tournés l'un vers l'autre.
